# EUROPEAN PATENT APPLICATION

(11) **EP 2 711 007 A1**
(43) Date of publication of application: **26.03.2014**
(21) Application number: 12382364.3
(22) Date of filing: 19.09.2012
(51) Int. Cl.: A61K 31/519, A61K 31/52, A61K 45/06, A61K 31/7064, A61K 31/7072, A61K 31/7076, A61P 35/00, A61P 35/04

(54) **4-Aminopyrazolo[3,4-d]pyrimidine for use in treating or preventing primary and metastatic breast and prostate cancer**

(71) Applicant: Institut Univ. de Ciència i Tecnologia, S.A., 08100 Mollet del Vallès Barcelona (ES); Pharmatest Services Ltd., 20520 Turku (FI)
(72) Inventor: Pascual Gilabert, Marta, 08100 Mollet del Vallès (ES); Castells Boliart, Josep, 08100 Mollet del Vallès (ES); López Gómez, Cristina, 08100 Mollet del Vallès (ES); Montilla Arevalo, Rafael, 08100 Mollet del Vallès (ES); Kytömaa, Riikka Annika, FI-20520 Turku (FI); Seppänen, Jani Antero, FI-20520 Turku (FI); Tuomela, Johanna Mirjami, FI-20520 Turku (FI); Vuorikoski, Heikki Johannes, FI-20520 Turku (FI); Halleen, Jussi Marko Juhani, FI-20520 Turku (FI)
(74) Representative: Oficina Ponti, SLP

(57) **Abstract**

The present invention relates to the field of biotechnology. The present invention relates to 4-aminopyrazolo[3,4-*d*]pyrimidine, i.e. a compound of formula (I) for use in the treatment or prevention of primary and metastatic breast and prostate cancer. The present invention also relates to a combination of a compound of formula (I) with at least one further compound for use in the treatment or prevention of primary and metastatic breast and prostate cancer. The present invention further relates to a pharmaceutical composition comprising the compound of formula (I) or the combination.

## Description

### Field of invention

The present invention relates to the field of biotechnology. In particular, the present invention relates to the field of cancer treatment. More in particular, the present invention relates to compounds for use in the treatment or prevention of primary and metastatic breast and prostate cancer.

### Background of the invention

Cancer, the uncontrolled growth of cells, is a major cause of death throughout the world. According to current World Health Organization data (http://www.who.int/cancer/en/), about 7.600.000 people worldwide died from cancer in 2008, representing almost 13% of total deaths caused by the disease. In fact, worldwide cancer deaths are projected to continue rising, with 11 million estimated deaths in 2030. Healthcare providers demand efficacious and well-tolerated therapies.

The foremost reason for the immense attention gained by the cancer therapy segment is the escalating prevalence of this disease as well as the huge number of needs that are yet to be met in the field of cancer treatment.

Among current treatments, chemotherapeutic compounds are extensively applied. However, in spite of their demonstrated efficacy, toxicity is still a huge problem that remains unsolved, since while providing some relief, anticancer drugs also cause serious side effects to patients. Many of the adverse effects of antineoplastics are an extension of their therapeutic action, which is not selective for malignant cells but affects all rapidly-dividing cells. Then, treatments must be carefully dosaged and timed.

It has been disclosed that there are substantial short- and long-term side effects from chemotherapy. Short-term effects typically occur during the course of treatment and generally resolve within months of the completion of therapy. In contrast, long-term effects can have a later onset and sustained impact- often lasting for many years. Moreover, there is also considerable variability in side effect profile across individuals (Partidge, A.H. J. of the National Cancer Institute Monographs, 2001, 30:135-142).

There are several known acute effects of antineoplastic drugs, often including nausea and vomiting, sometimes extremely severe. In addition, many of these compounds are irritant or vesicant, and produce local pain, irritation, and inflammation at the administration site; extravasation may lead to ulceration and necrosis. Adverse effects may be expected in tissues where normal cell division is fairly rapid, e.g. the bone marrow, lymphoreticular tissue, gastrointestinal mucosa, skin, and gonads, as well as in the fetus. The most common serious effect is bone-marrow depression and immunosupression, causing patients great risk of severe and disseminated infections (Martindale, The Complete Drug Reference, 36th Ed. - Antineoplastics monography). In very long term treatments, patients may even develop secondary malignancies, suggesting that anticancer drugs may themselves be carcinogenic, as well.

Dosage of chemotherapeutic products is a problematic matter, as well: when the dose is too low, it might be ineffective against the tumour, whereas, at excessive doses, the toxicity (i.e. side effects) is usually intolerable for patients. Hence, pharma companies around the world make efforts to formulate drugs enclosing maximum efficacy and minimal side effects.

Moreover, there are several known data indicating that, as cancer progresses, heterogeneity increases and multiple mechanisms may account for the drug resistance, even in the same patient (Madan et al, Clin. Cancer Res. 2011, 17(2), 3892-3902; Hung et al, Cancer Lett. 2012, 320:138-149).

Among the most known types of cancer, breast and prostate cancer are especially significant in women and men, respectively.

Breast cancer is one of the most common human neoplasms, and by far the most frequent cancer among women with an estimated 1.38 million new cancer cases diagnosed in 2008 (23% of all cancers), and ranks second overall (10.9% of all cancers). It is now the most common cancer both in developed and developing regions with an estimated 458500 female deaths in 2008 or nearly one in seven (around 14%) of all cancer deaths in women. Also, breast cancer affects men, but it is rare: e.g. estimation for 2012 in US is 410 deaths in men. (Data extracted from IARC: International Agency for Research on Cancer, WHO; http://www.iarc.fr/en/cancertopics/breasttopics.php and Globocan 2008 report).

Prostate cancer contributes significantly to the overall cancer burden, being second most frequently diagnosed cancer of men (899000 new cases, 13.6% of the total) and the fifth most common cancer overall. Nearly three-quarters of the registered cases occur in developed countries (644000 cases). With an estimated 258 000 deaths in 2008, prostate cancer is the sixth leading cause of death from cancer in men (6.1% of the total). (Data extracted from IARC: International Agency for Research on Cancer, WHO; http://www.iarc.fr/en/cancertopics/prostatetopics.php and Globocan 2008 report).

The two main group of cytotoxic drugs used in the treatment of malignant diseases are the alkylating agents and the antimetabolites. The antimetabolites are a class of drugs that inhibit DNA synthesis either directly or through inhibition of DNA precursor synthesis on *de novo* or salvage pathways (Galmarini et al, Lancet Oncol. 2002, 3: 415-424). These actions result in the induction of apoptotic cell death.

Antimetabolites may be subdivided into folic acid and nucleoside analogs (purine or pyrimidine) antagonists. Nucleoside analogs comprise a major class of chemotherapeutic agents for the treatment of cancer and viral diseases. The effectiveness of these agents as antitumor drugs was established in the 1960s with the nucleobase antimetabolite 5-fluorouracil (Heidelberg et al, Adv. Enzymol. Relat. Areas Mol. Biol. 1983, 54: 58-119).

A major obstacle associated with the use of these drugs is the lack of selectivity towards cancerous cells, and consequently, the effective doses are particularly high. Hence, toxicity becomes the main limiting factor to the treatment.

Moreover, chemoresistance to some of the unique standards of care (such as gemcitabine in pancreatic cancer) is a major scientific problem that is still an unmet need (Wai Hung et al, Cancer Lett. 2012, 320: 138-149).

However, one of the remarkable features that remains unexplained about nucleobase and nucleoside analogs is how drugs with such similar structural features, which share metabolic pathways and elements of their mechanisms of action, show such diversity in their clinical activities (Sampath, D. et al, Oncogene 2003, 22, 9063-9074).

In view of the prior art, there is still the need for finding new drugs or combinations of drugs that could prove more effective than the known single-agent or combination regimens, while maintaining an acceptable or improved toxicity profile.

The present inventors have surprisingly found that a compound of formula (I) (also known as 4-Aminopyrazolo[3,4-*d*]pyrimidine, Pyrazolo[3,4-*d*]pyrimidin-4-amine or 4APP) is effective in breast and prostate cancer therapy by itself and when administered in combination with several other compounds, even a synergistic antiproliferative effect is surprisingly obtained as compared to the effect of these compounds alone.

The adenine analogue, Pyrazolo[3,4-*d*]pyrimidin-4-amine was synthesized by Robins in 1956 (Robins et al, JACS, 1956, 78(4), 784-789), and it has proved to affect nucleotide synthesis, whose inhibition is related to cytotoxic activity of the above mentioned compound.

Since then, several studies have been published related to biological activity of 4APP.

4APP has been reported as an agent to prevent the bulking of activated sludge in wastewater treatment (JP 05309388), a hypolipidemic drug (Belloni et al, Histology and Histopathology, 1989, 4 (1), 31-37; Feingold et al, Diabetes, 1987, 36 (11), 1223-1229), a trypanostatic agent (*Trypanosoma rangeli*: Avila et al, Comp. Biochem. Phys., Part C: Pharm, Tox. & Endocr. 1986, 83C(2), 291-294; *Trypanosoma cruzi*: Avila et al, Curr. Chemother. Immunother. Proc. Int. Congr. Chemother., 12th, 1982, 1:131-133), a inhibitor of *Crithidia fasciculate* (Dewey et al, Can. J. Biochem. 1977, 55(1), 110-112), a inhibitor of *Candida utilis* (Brecher et al, Biochem. Pharmacology, 1962, 11:1101-1107), an immunostimulant for neoplasm treatment (JP 62029524, abstract), an inhibitor of Shiga toxin 1 produced by enterohaemorrhagic *E. coli* strains in the pathogenesis of haemorrhagic colitis (Brigotti et al, Nuc. Ac. Res. 2000, 28 (12), 2383-2388) and as a inhibitor of xanthine oxidase to treat gout (Hsieh et al, Bioorg. Med. Chem., 2007, 15:3450-3456).

Hsu et al (Science, 1956, 123:848-849) disclose the study of antineoplastic action *in vitro* for 4APP in human cervical carcinoma, B-3 adenocarcinoma of mice, skin and heart tissues of mouse embryos, mouse renal papillae, and human preputial skin. However, authors conclude that tissues showed relatively indifferent response to the treatment with 4APP at all concentrations tested.

Tarnowski et al (Cancer Res. 1958, 18:1-48) disclose cancer chemotherapy screening on RC and S790 mouse mammary carcinomas. A set of chemicals, including 4APP were arbitrarily selected. However, according to authors' results, none tested compounds, except thioguanine, 6-methyl-mercaptopurine, purine hydrochloride, fumagillin, actidione, 6-diazo-5-oxo-L-norleucine, puromycin and *N,N',N"-*triethylenethiophosphoramidine were found to be capable of producing 50% or more tumor growth inhibition. 4APP showed only 10% inhibition in those conditions, which was considered non active (Henderson et al, Cancer Res. 1960, 20:1618-1624).

Skipper et al (Proc. Soc. Exper. Biol. & Med., 1955, 89:594-596; Cancer Res. 1957, 17:579-596) disclose potent antitumour activity against Adenocarcinoma 755, Leukemia L5178, moderate activity in Leukemia L1210. Moderate activity was also reported by Sugiura et al (Ann. New York Acad. Sci., 1958, 76:575-585) in Ehrlich carcinoma, and little or no activity was reported by the same authors against Dunning leukaemia and Sarcoma 180. In fact, according to Henderson (Henderson et al, Cancer Res. 1960, 20:1618-1624), 4APP had no therapeutic effect on the few human tumours treated thus far.

Shaw et al (Cancer, 1960, 13(3): 482-489) disclose animal and man toxicity and metabolism of 4APP. Authors state that due to its purinic nature, the major interest in the use of this compound in cancer chemotherapy centers about the treatment of acute leukaemia. However, although up to 9 patients were treated with the compound, it did not demonstrate any antitumour activity in man.

Pharmacological studies have been disclosed in animals and men. Scholler et al (Proc. Soc. Exp. Biol. Med., 1956, 93:398-402) performed toxicological and pathological studies in mice, rats, cats and dogs, where it was detected a fatty change in the liver neither associated with necrosis nor with inflammation.

It is increasingly being realized that effective treatment of cancer not only requires efficient drugs but also optimal use of specific combination of anticancer drugs. Combination therapy is increasingly being considered as a better alternative to monotherapy in the treatment of cancer. The sequential or simultaneous use of two or more drugs is termed as combination therapy.

Various combinations of chemotherapeutic agents have been discussed in prior art. However, combinations generally consist of previously known oncolytic agents, including Gemcitabine, Capecitabine or related marketed anticancer drug compounds (WO0018393, WO2004041308, WO03043632, US20110312528). Gemcitabine (2',2'-difluorodeoxycytidine) is currently marketed as Gemzar® by Eli Lilly, as monohydrochloride salt in the β-isomer form. It is a nucleoside analogue of deoxycytidine, which was first disclosed in US Pat. No. 4,808,614 and US Pat No. 5,464,826. Gemcitabine has been indicated as the first-line therapy for locally advanced (nonresectable Stage II or II) or metastatic (Stage IV) adenocarcinoma of the pancreas. Gemcitabine has also been indicated as a second-line therapy for patients who have previously been treated with fluorouracil.

The present inventors have surprisingly found that 4APP exhibits an extremely potent antiproliferative activity in breast and prostate cancer, in comparison to known marketed anticancer agents Gemcitabine and Floxuridine, while at the same time not affecting the normal cell division of certain non tumour cells, *i.e.* it exhibits no toxic effect on this type of normal cells.

Also surprisingly, when using the compound of formula (I) (4APP) for studying other type of cancer cells such as pancreatic cancer cells, no effect was detected.

In addition, the present inventors have also surprisingly found that certain combinations of 4APP with a further molecule, not necessarily known in prior art as therapeutic or even as anticancer drugs, exhibit antiproliferative synergistic effect in the same solid tumours (breast and prostate cancer) in comparison to Gemcitabine and Floxuridine, while at the same time not affecting the normal cell division of certain non tumorous cells, *i.e.* the combination does not exhibit toxic effects on this type of normal cells.

As in the case of using the compound of formula (I) alone, no effect was detected when other type of cancer cells such as pancreatic cancer cells were studied.

### Summary of the invention

A first object of the present invention relates to a compound of formula (I) for use in the treatment or prevention of primary and metastatic breast and prostate cancer.

A second object of the present invention relates to a combination of a compound of formula (I) with at least one further compound for use in the treatment or prevention of primary and metastatic breast and prostate cancer.

A third object of the present invention relates to a pharmaceutical composition comprising the compound of formula (I) or the combination according the second object of the invention.

### Brief description of the drawings

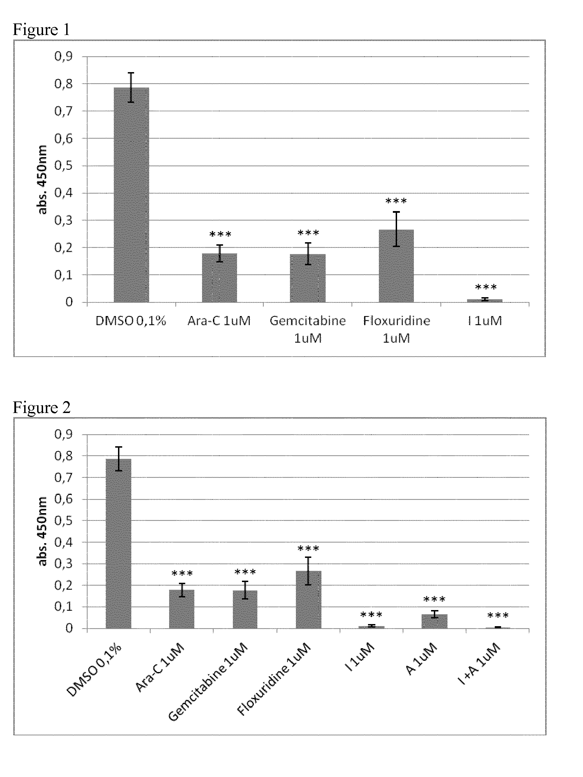

Figure 1 represents the effects of vehicle control, compound I and control nucleoside analogs Ara-C, gemcitabine and Floxuridine on proliferation of MCF-7 breast cancer cells after 5 days incubation (mean±SD). Statistical analysis was performed using ANOVA. As statistical differences were observed (p=0), the pairwise comparison against the vehicle control group was performed using Tukey test. Notation: *** = statistically significant difference with p-value < 0.001, ** = p-value < 0.01, * = p-value < 0.05, and NS = Non-Significant.
Figure 2 represents the effects of vehicle control, compounds I and A (see table 1), combination of compounds I and A, and control nucleoside analogs Ara-C, gemcitabine and Floxuridine on proliferation of MCF-7 breast cancer cells after 5 days incubation (mean±SD). Statistical analysis was performed using ANOVA. As statistical differences were observed (p=0), the pairwise comparison against the vehicle control group was performed using Tukey test. Notation: *** = statistically significant difference with p-value < 0.001, ** = p-value < 0.01, * = p-value < 0.05, and NS = Non-Significant.

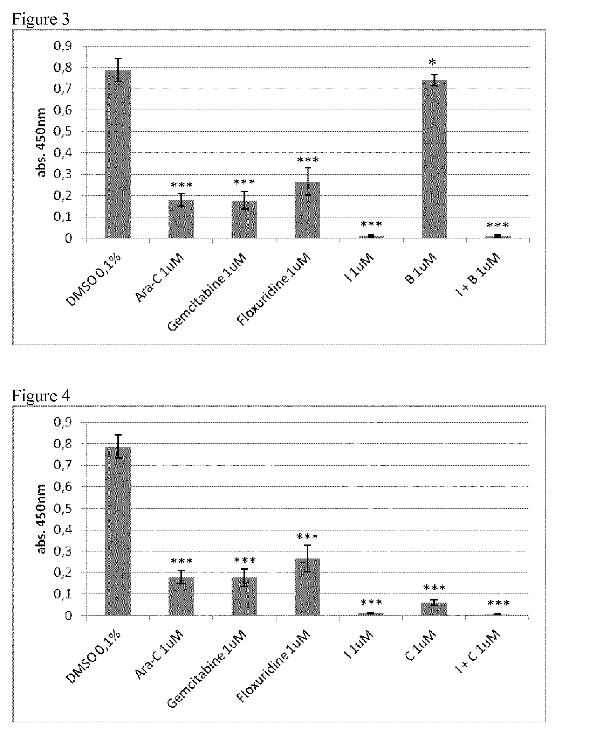

Figure 3 represents the effects of vehicle control, compounds I and B (see table 1), combination of compounds I and B and control nucleoside analogs Ara-C, gemcitabine and Floxuridine on proliferation of MCF-7 breast cancer cells after 5 days incubation (mean±SD). Statistical analysis was performed using ANOVA. As statistical differences were observed (p=0), the pairwise comparison against the vehicle control group was performed using Tukey test. Notation: *** = statistically significant difference with p-value < 0.001, ** = p-value < 0.01, * = p-value < 0.05, and NS = Non-Significant.
Figure 4 represents the effects of vehicle control, compounds I and C (see table 1), combination of compounds I and C and control nucleoside analogs Ara-C, gemcitabine and Floxuridine on proliferation of MCF-7 breast cancer cells after 5 days incubation (mean±SD). Statistical analysis was performed using ANOVA. As statistical differences were observed (p=0), the pairwise comparison against the vehicle control group was performed using Tukey test. Notation: *** = statistically significant difference with p-value < 0.001, ** = p-value < 0.01, * = p-value < 0.05, and NS = Non-Significant.

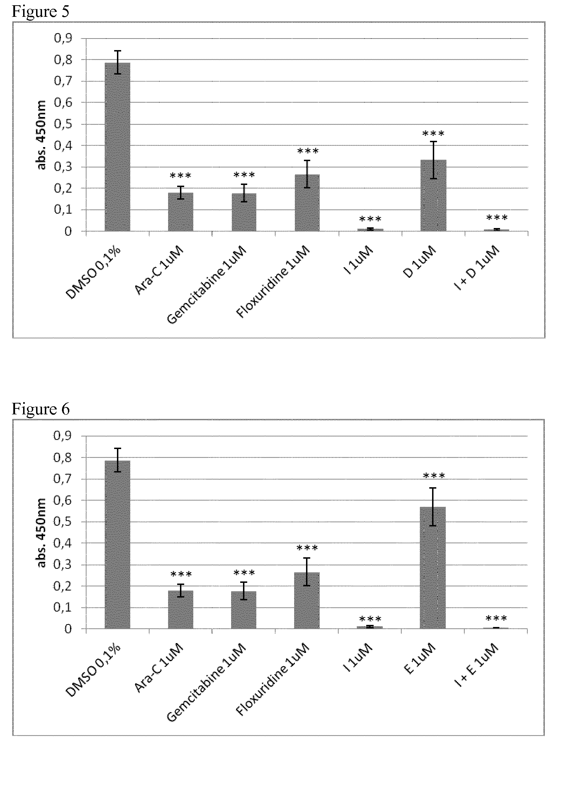

Figure 5 represents the effects of vehicle control, compounds I and D (see table 1), combination of compounds I and D and control nucleoside analogs Ara-C, gemcitabine and Floxuridine on proliferation of MCF-7 breast cancer cells after 5 days incubation (mean±SD). Statistical analysis was performed using ANOVA. As statistical differences were observed (p=0), the pairwise comparison against the vehicle control group was performed using Tukey test. Notation: *** = statistically significant difference with p-value < 0.001, ** = p-value < 0.01, * = p-value < 0.05, and NS = Non-Significant.
Figure 6 represents the effects of vehicle control, compounds I and E (see table 1), combination of compounds I and E and control nucleoside analogs Ara-C, gemcitabine and Floxuridine on proliferation of MCF-7 breast cancer cells after 5 days incubation (mean±SD). Statistical analysis was performed using ANOVA. As statistical differences were observed (p=0), the pairwise comparison against the vehicle control group was performed using Tukey test. Notation: *** = statistically significant difference with p-value < 0.001, ** = p-value < 0.01, * = p-value < 0.05, and NS = Non-Significant.

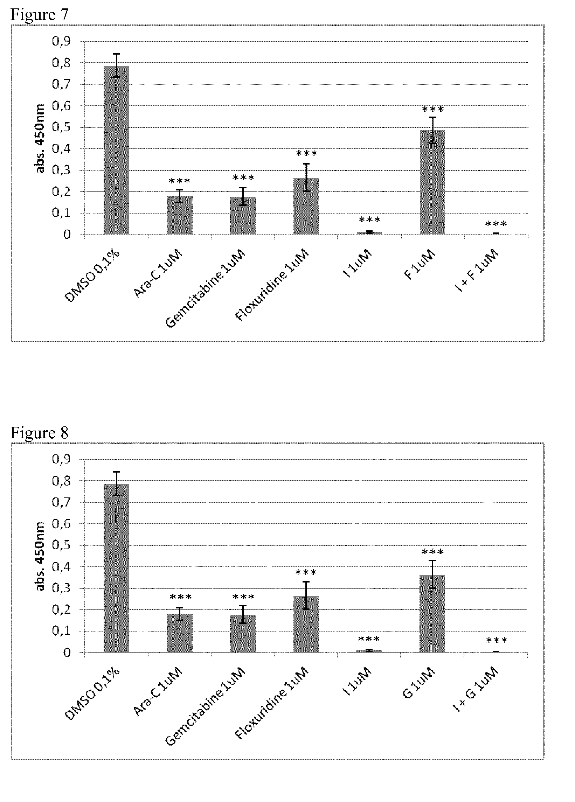

Figure 7 represents the effects of vehicle control, compounds I and F (see table 1), combination of compounds I and F and control nucleoside analogs Ara-C, gemcitabine and Floxuridine on proliferation of MCF-7 breast cancer cells after 5 days incubation (mean±SD). Statistical analysis was performed using ANOVA. As statistical differences were observed (p=0), the pairwise comparison against the vehicle control group was performed using Tukey test. Notation: *** = statistically significant difference with p-value < 0.001, ** = p-value < 0.01, * = p-value < 0.05, and NS = Non-Significant.
Figure 8 represents the effects of vehicle control, compounds I and G (see table 1), combination of compounds I and G and control nucleoside analogs Ara-C, gemcitabine and Floxuridine on proliferation of MCF-7 breast cancer cells after 5 days incubation (mean±SD). Statistical analysis was performed using ANOVA. As statistical differences were observed (p=0), the pairwise comparison against the vehicle control group was performed using Tukey test. Notation: *** = statistically significant difference with p-value < 0.001, ** = p-value < 0.01, * = p-value < 0.05, and NS = Non-Significant.

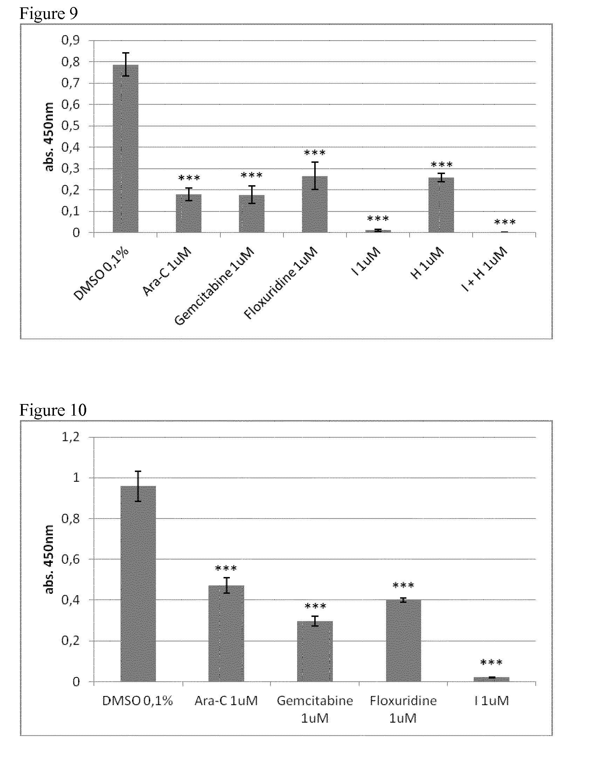

Figure 9 represents the effects of vehicle control, compounds I and H (see table 1), combination of compounds I and H and control nucleoside analogs Ara-C, gemcitabine and Floxuridine on proliferation of MCF-7 breast cancer cells after 5 days incubation (mean±SD). Statistical analysis was performed using ANOVA. As statistical differences were observed (p=0), the pairwise comparison against the vehicle control group was performed using Tukey test. Notation: *** = statistically significant difference with p-value < 0.001, ** = p-value < 0.01, * = p-value < 0.05, and NS = Non-Significant.
Figure 10 represents the effects of vehicle control, compound I and control nucleoside analogs Ara-C, gemcitabine and Floxuridine on proliferation of LNCaP prostate cancer cells after 5 days incubation (mean±SD). Statistical analysis was performed using ANOVA. As statistical differences were observed (p=0), the pairwise comparison against the vehicle control group was performed using Tukey test. Notation: *** = statistically significant difference with p-value < 0.001, ** = p-value < 0.01, * = p-value < 0.05, and NS = Non-Significant.

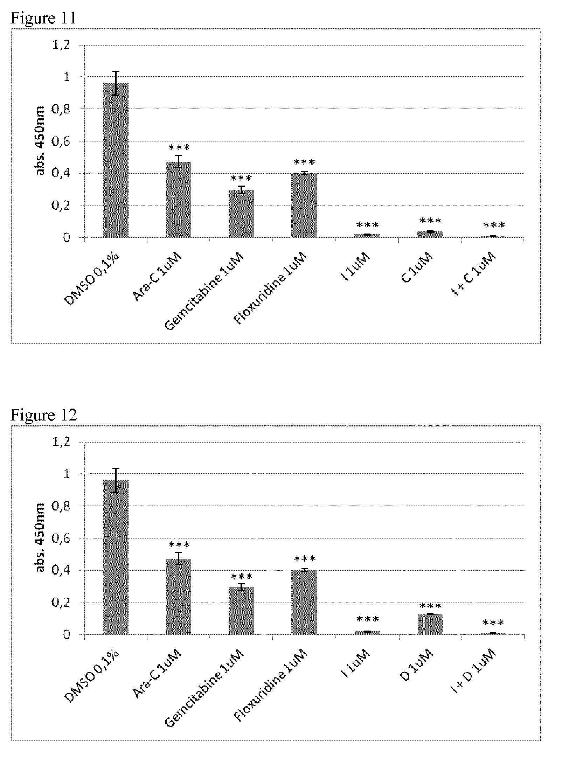

Figure 11 represents the effects of vehicle control, compounds I and C (see table 1), combination of compounds I and C and control nucleoside analogs Ara-C, gemcitabine and Floxuridine on proliferation of LNCaP prostate cancer cells after 5 days incubation (mean±SD). Statistical analysis was performed using ANOVA. As statistical differences were observed (p=0), the pairwise comparison against the vehicle control group was performed using Tukey test. Notation: *** = statistically significant difference with p-value < 0.001, ** = p-value < 0.01, * = p-value < 0.05, and NS = Non-Significant.
Figure 12 represents the effects of vehicle control, compounds I and D (see table 1), combination of compounds I and D and control nucleoside analogs Ara-C, gemcitabine and Floxuridine on proliferation of LNCaP prostate cancer cells after 5 days incubation (mean±SD). Statistical analysis was performed using ANOVA. As statistical differences were observed (p=0), the pairwise comparison against the vehicle control group was performed using Tukey test. Notation: *** = statistically significant difference with p-value < 0.001, ** = p-value < 0.01, * = p-value < 0.05, and NS = Non-Significant.

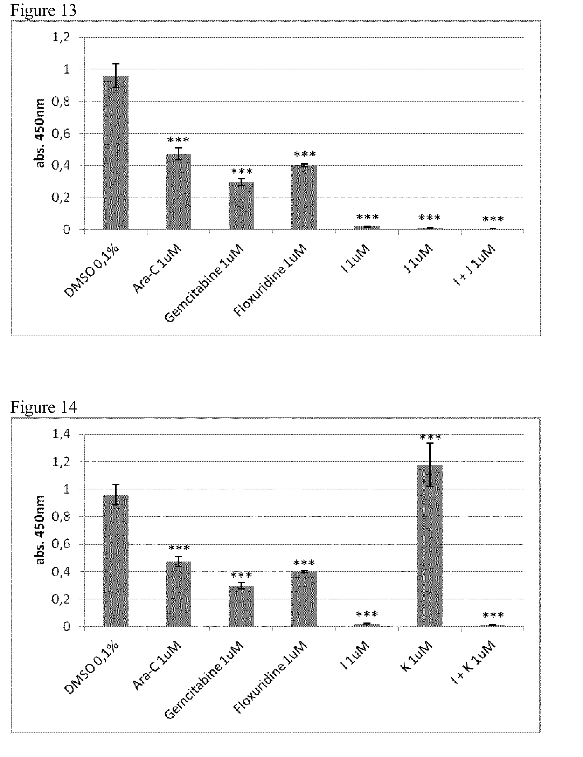

Figure 13 represents the effects of vehicle control, compounds I and J (see table 1), combination of compounds I and J and control nucleoside analogs Ara-C, gemcitabine and Floxuridine on proliferation of LNCaP prostate cancer cells after 5 days incubation (mean±SD). Statistical analysis was performed using ANOVA. As statistical differences were observed (p=0), the pairwise comparison against the vehicle control group was performed using Tukey test. Notation: *** = statistically significant difference with p-value < 0.001, ** = p-value < 0.01, * = p-value < 0.05, and NS = Non-Significant.
Figure 14 represents the effects of vehicle control, compounds I and K (see table 1), combination of compounds I and K and control nucleoside analogs Ara-C, gemcitabine and Floxuridine on proliferation of LNCaP prostate cancer cells after 5 days incubation (mean±SD). Statistical analysis was performed using ANOVA. As statistical differences were observed (p=0), the pairwise comparison against the vehicle control group was performed using Tukey test. Notation: *** = statistically significant difference with p-value < 0.001, ** = p-value < 0.01, * = p-value < 0.05, and NS = Non-Significant.

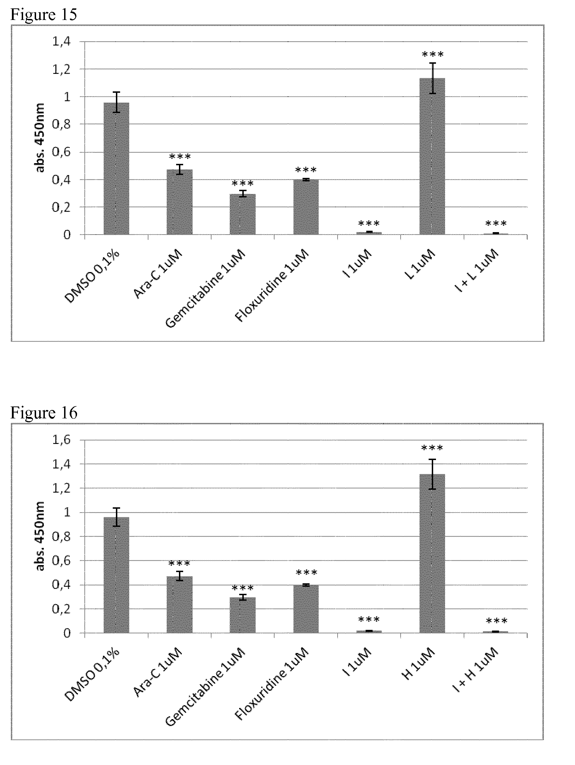

Figure 15 represents the effects of vehicle control, compounds I and L (see table 1), combination of compounds I and L and control nucleoside analogs Ara-C, gemcitabine and Floxuridine on proliferation of LNCaP prostate cancer cells after 5 days incubation (mean±SD). Statistical analysis was performed using ANOVA. As statistical differences were observed (p=0), the pairwise comparison against the vehicle control group was performed using Tukey test. Notation: *** = statistically significant difference with p-value < 0.001, ** = p-value < 0.01, * = p-value < 0.05, and NS = Non-Significant.
Figure 16 represents the effects of vehicle control, compounds I and H (see table 1), combination of compounds I and H and control nucleoside analogs Ara-C, gemcitabine and Floxuridine on proliferation of LNCaP prostate cancer cells after 5 days incubation (mean±SD). Statistical analysis was performed using ANOVA. As statistical differences were observed (p=0), the pairwise comparison against the vehicle control group was performed using Tukey test. Notation: *** = statistically significant difference with p-value < 0.001, ** = p-value < 0.01, * = p-value < 0.05, and NS = Non-Significant.

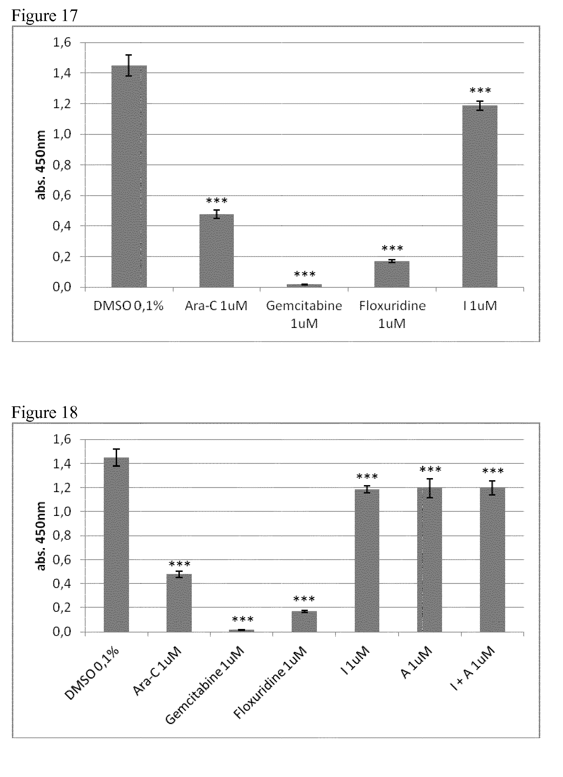

Figure 17 represents the effects of vehicle control, compound I and control nucleoside analogs Ara-C, gemcitabine and Floxuridine on proliferation of PNT1A immortalized normal prostate cells after 5 days incubation (mean±SD). Statistical analysis was performed using ANOVA. As statistical differences were observed (p=0), the pairwise comparison against the vehicle control group was performed using Tukey test. Notation: *** = statistically significant difference with p-value < 0.001, ** = p-value < 0.01, * = p-value < 0.05, and NS = Non-Significant.
Figure 18 represents the effects of vehicle control, compounds I and A (see table 1), combination of compounds I and A, and control nucleoside analogs Ara-C, gemcitabine and Floxuridine on proliferation of PNT1A immortalized normal prostate cells after 5 days incubation (mean±SD). Statistical analysis was performed using ANOVA. As statistical differences were observed (p=0), the pairwise comparison against the vehicle control group was performed using Tukey test. Notation: *** = statistically significant difference with p-value < 0.001, ** = p-value < 0.01, * = p-value < 0.05, and NS = Non-Significant.

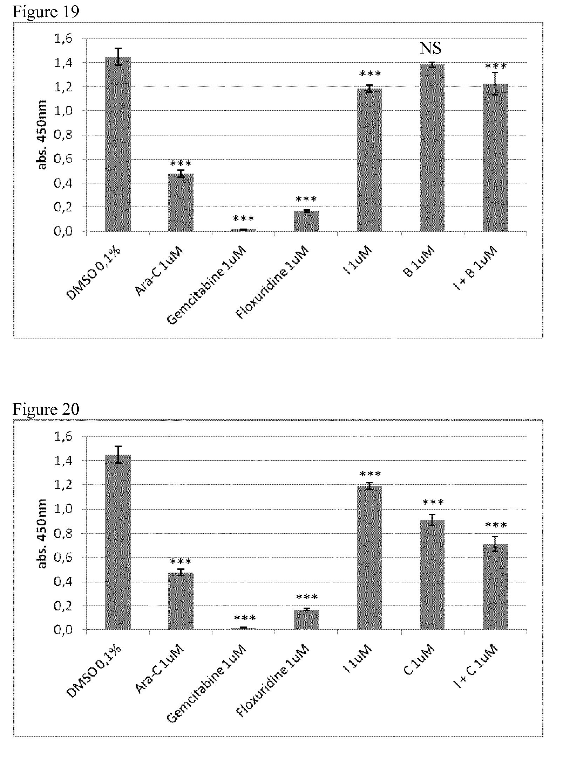

Figure 19 represents the effects of vehicle control, compounds I and B (see table 1), combination of compounds I and B and control nucleoside analogs Ara-C, gemcitabine and Floxuridine on proliferation of PNT1A immortalized normal prostate cells after 5 days incubation (mean±SD). Statistical analysis was performed using ANOVA. As statistical differences were observed (p=0), the pairwise comparison against the vehicle control group was performed using Tukey test. Notation: *** = statistically significant difference with p-value < 0.001, ** = p-value < 0.01, * = p-value < 0.05, and NS = Non-Significant.
Figure 20 represents the effects of vehicle control, compounds I and C (see table 1), combination of compounds I and C and control nucleoside analogs Ara-C, gemcitabine and Floxuridine on proliferation of PNT1A immortalized normal prostate cells after 5 days incubation (mean±SD). Statistical analysis was performed using ANOVA. As statistical differences were observed (p=0), the pairwise comparison against the vehicle control group was performed using Tukey test. Notation: *** = statistically significant difference with p-value < 0.001, ** = p-value < 0.01, * = p-value < 0.05, and NS = Non-Significant.

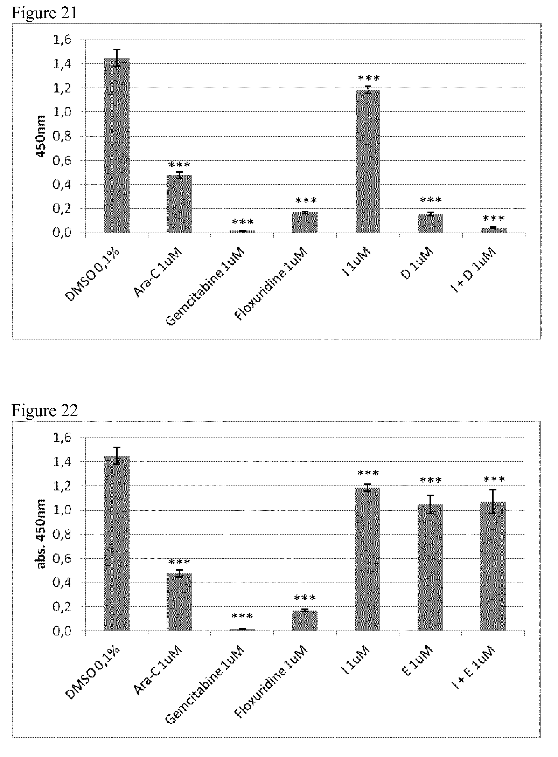

Figure 21 represents the effects of vehicle control, compounds I and D (see table 1), combination of compounds I and D and control nucleoside analogs Ara-C, gemcitabine and Floxuridine on proliferation of PNT1A immortalized normal prostate cells after 5 days incubation (mean±SD). Statistical analysis was performed using ANOVA. As statistical differences were observed (p=0), the pairwise comparison against the vehicle control group was performed using Tukey test. Notation: *** = statistically significant difference with p-value < 0.001, ** = p-value < 0.01, * = p-value < 0.05, and NS = Non-Significant.
Figure 22 represents the effects of vehicle control, compounds I and E (see table 1), combination of compounds I and E and control nucleoside analogs Ara-C, gemcitabine and Floxuridine on proliferation of PNT1A immortalized normal prostate cells after 5 days incubation (mean±SD). Statistical analysis was performed using ANOVA. As statistical differences were observed (p=0), the pairwise comparison against the vehicle control group was performed using Tukey test. Notation: *** = statistically significant difference with p-value < 0.001, ** = p-value < 0.01, * = p-value < 0.05, and NS = Non-Significant.

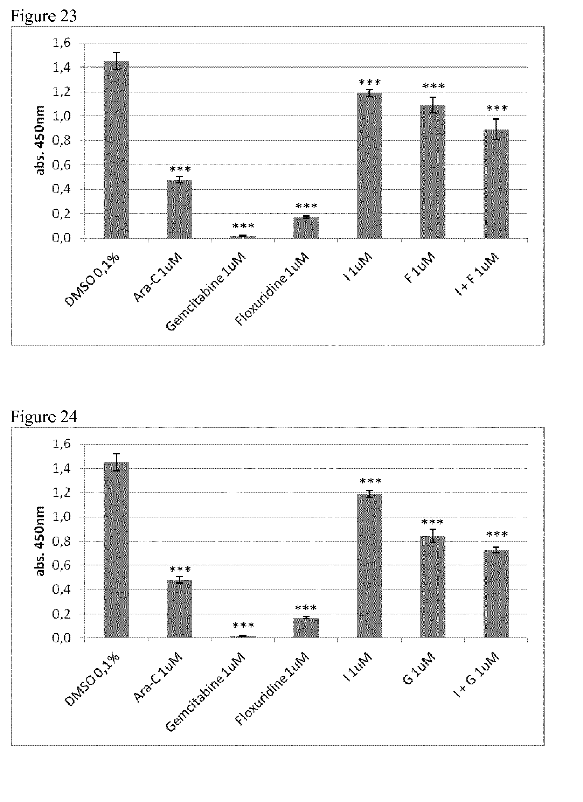

Figure 23 represents the effects of vehicle control, compounds I and F (see table 1), combination of compounds I and F and control nucleoside analogs Ara-C, gemcitabine and Floxuridine on proliferation of PNT1A immortalized normal prostate cells after 5 days incubation (mean±SD). Statistical analysis was performed using ANOVA. As statistical differences were observed (p=0), the pairwise comparison against the vehicle control group was performed using Tukey test. Notation: *** = statistically significant difference with p-value < 0.001, ** = p-value < 0.01, * = p-value < 0.05, and NS = Non-Significant.
Figure 24 represents the effects of vehicle control, compounds I and G (see table 1), combination of compounds I and G and control nucleoside analogs Ara-C, gemcitabine and Floxuridine on proliferation of PNT1A immortalized normal prostate cells after 5 days incubation (mean±SD). Statistical analysis was performed using ANOVA. As statistical differences were observed (p=0), the pairwise comparison against the vehicle control group was performed using Tukey test. Notation: *** = statistically significant difference with p-value < 0.001, ** = p-value < 0.01, * = p-value < 0.05, and NS = Non-Significant.

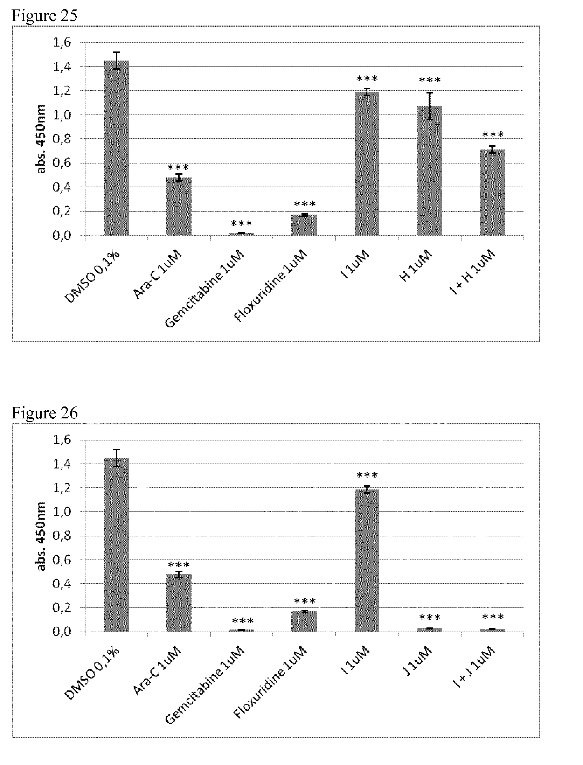

Figure 25 represents the effects of vehicle control, compounds I and H (see table 1), combination of compounds I and H and control nucleoside analogs Ara-C, gemcitabine and Floxuridine on proliferation of PNT1A immortalized normal prostate cells after 5 days incubation (mean±SD). Statistical analysis was performed using ANOVA. As statistical differences were observed (p=0), the pairwise comparison against the vehicle control group was performed using Tukey test. Notation: *** = statistically significant difference with p-value < 0.001, ** = p-value < 0.01, * = p-value < 0.05, and NS = Non-Significant.
Figure 26 represents the effects of vehicle control, compounds I and J (see table 1), combination of compounds I and J and control nucleoside analogs Ara-C, gemcitabine and Floxuridine on proliferation of PNT1A immortalized normal prostate cells after 5 days incubation (mean±SD). Statistical analysis was performed using ANOVA. As statistical differences were observed (p=0), the pairwise comparison against the vehicle control group was performed using Tukey test. Notation: *** = statistically significant difference with p-value < 0.001, ** = p-value < 0.01, * = p-value < 0.05, and NS = Non-Significant.

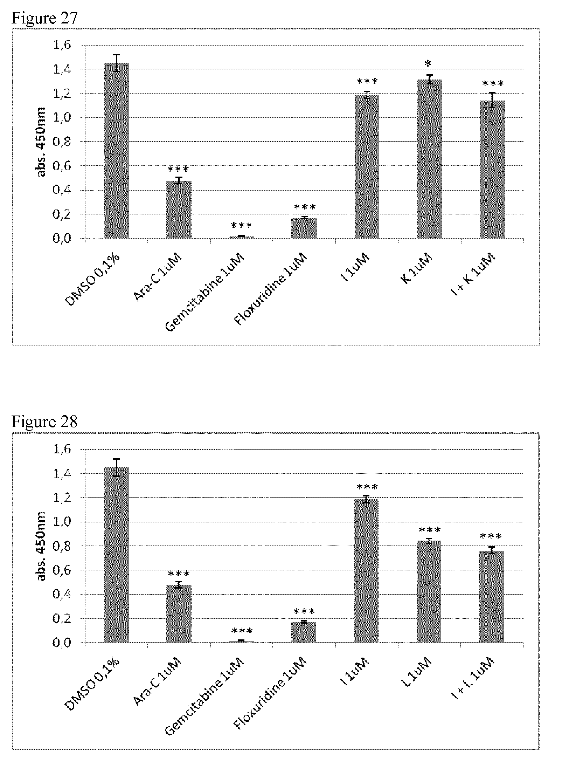

Figure 27 represents the effects of vehicle control, compounds I and K (see table 1), combination of compounds I and K and control nucleoside analogs Ara-C, gemcitabine and Floxuridine on proliferation of PNT1A immortalized normal prostate cells after 5 days incubation (mean±SD). Statistical analysis was performed using ANOVA. As statistical differences were observed (p=0), the pairwise comparison against the vehicle control group was performed using Tukey test. Notation: *** = statistically significant difference with p-value < 0.001, * * = p-value < 0.01, * = p-value < 0.05, and NS = Non-Significant.
Figure 28 represents the effects of vehicle control, compounds I and L (see table 1), combination of compounds I and L and control^{*} nucleoside analogs Ara-C, gemcitabine and Floxuridine on proliferation of PNT1A immortalized normal prostate cells after 5 days incubation (mean±SD). Statistical analysis was performed using ANOVA. As statistical differences were observed (p=0), the pairwise comparison against the vehicle control group was performed using Tukey test. Notation: *** = statistically significant difference with p-value < 0.001, ** = p-value < 0.01, * = p-value < 0.05, and NS = Non-Significant.

### Detailed description of the invention

In a first aspect, the present invention relates to a compound of formula (I) 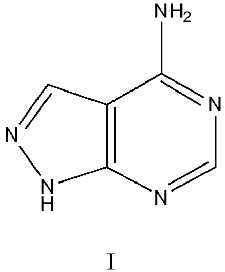 also known as 4-Aminopyrazolo[3,4-*d*]pyrimidine; Pyrazolo[3,4-*d*]pyrimidin-4-amine or 4APP; or a tautomer, solvate, hydrate or pharmaceutically acceptable salt thereof for use in the treatment or prevention of primary and metastatic breast cancer.

The present invention also relates to a compound of formula (I) 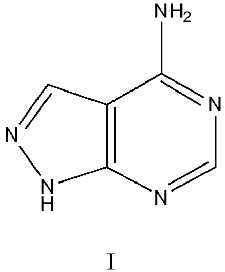 also known as 4-Aminopyrazolo[3,4-*d*]pyrimidine; Pyrazolo[3,4-*d*]pyrimidin-4-amine or 4APP; or a tautomer, solvate, hydrate or pharmaceutically acceptable salt thereof for use in the treatment or prevention of primary and metastatic prostate cancer.

In the present invention, the terms "compound of this invention", "compound of the present invention", "compound of formula (I)", "compound I", "4APP", "Pyrazolo[3,4-*d*]pyrimidin-4-amine" and "4-Aminopyrazolo[3,4-*d*]pyrimidine" include the compound itself, tautomers, solvates, hydrates or pharmaceutically acceptable salts thereof.

In a second aspect, the present invention relates to a combination of the compound of formula (I) or a tautomer, solvate, hydrate or a pharmaceutically acceptable salt thereof with at least one further compound (being or not chemotherapeutic) for use in the treatment or prevention of primary and metastatic breast cancer. Also, the present invention relates to a combination of the compound of formula (I) or a tautomer, solvate, hydrate or a pharmaceutically acceptable salt thereof with at least one further compound (being or not chemotherapeutic) for use in the treatment or prevention of primary and metastatic prostate cancer.

The effect of said combination is synergistic when compared to the effect of any of the components of said combination alone.

The combination of the compound of formula (I) and the at least one further compound, may be administered concurrently, sequentially, or as part of the same composition.

When the at least one further compound is a known therapeutic agent to be used together with the compound of formula (I), this therapeutic agent is defined as a compound which is known as effective for the prevention or treatment of diseases or disorders, such as cancer or associated conditions as viral infections. Optionally, the at least one further compound may not be known as therapeutic agent up to date, or at least, not being known as a chemotherapeutic agent up to date.

The at least one further compound to be used together with the compound of formula (I) may include, but are not limited to, chemotherapeutic agents, such as alkylating agents, antimetabolites (including nucleoside analogs and nucleobases), spindle poison plant alkaloids, cytotoxic/antitumour antibiotics, topoisomerase inhibitors, antibodies, photosensitizers, kinase inhibitors; anti-hormonal agents; aromatase inhibitors; anti-androgens; ribozymes; vaccines such as gene therapy vaccines; anti-angiogenic agents, and antiviral agents. Chemotherapeutic agents include compounds used in targeted therapy and conventional chemotherapy. Exemplary chemotherapeutic agents include, but are not limited to: 5-FU (fluorouracil, 5-fluorouracil, CAS No. 51-24-8), gemcitabine (GEMZAR®, Eli Lilly), paclitaxel (TAXOL, Bristol-Myers Squibb Oncology), capecitabine (XELODA®, Roche), cis-platin, mitoxantrone, etoposide, camptothecin, vinblastine, paclitaxel, docetaxel, mithramycin A, dexamethasone, caffeine and pharmaceutically acceptable salts, acids and derivatives of any of the above.

As further used herein, the term "nucleoside" refers to all compounds in which a heterocyclic base is covalently coupled to a sugar, and especially preferred coupling of the nucleoside to the sugar includes a Cl'-(glycosidic) bond of a carbon atom in a sugar to a carbon- or heteroatom (typically nitrogen) in the heterocyclic base.

The term "nucleoside analogue" as used herein refers to all nucleosides in which the sugar may not be a ribofuranose or deoxyribofuranose and/or in which the heterocyclic base is not a naturally occurring base (e.g., A, G, C, T, I, etc.). Nucleoside analogs may be therapeutic compounds, such as anticancer, antiviral or antifungal agents, and even non therapeutic compounds, such as plant hormones. Examples of nucleoside analogs include, but are not limited to, 5-fluoro-2'-deoxyuridine, 5-chloro-2'-deoxyuridine, 5-bromo-2'-deoxyuridine, 5-iodo-2'-deoxyuridine, 5-fluorouridine, 5-chlorouridine, 5-bromouridine, 5-iodouridine, 6-methylpurine-2'-deoxyriboside, 6-methylpurine riboside, 2-fluoro-2'-deoxyadenosine, 2-fluoroadenosine, trifluorothymidine, 5-bromovinyl-2'-deoxyuridine, *N*6, *N*6-dimethyladenosine, 6-mercaptoguanosine, 8-aza-7-deaza-2'-deoxyadenosine, *trans*-zeatin riboside, *trans-*zeatin deoxyriboside, 5-vinyl-2'-deoxyuridine, 2-amino-6-bromopurine riboside, 2-amino-6-bromopurine deoxyriboside and 6-(ethylthio)inosine. More preferably, the nucleoside analog is selected from 5-bromo-2'-deoxyuridine, 5-iodo-2'-deoxyuridine, 2-fluoro-2'-deoxyadenosine, *N*6,*N*6-dimethyladenosine, 8-aza-7-deaza-2'-deoxyadenosine, 2-amino-6-bromopurine deoxyriboside or 6-(ethylthio)inosine.

The term "sugar" refers to all carbohydrates, stereoisomers and derivatives thereof, wherein particularly contemplated derivatives include deletion, substitution or addition or a chemical group or atom in the sugar. For example, especially contemplated deletions include 2'-deoxy and/or 3'-deoxy sugars. Especially contemplated substitutions include replacement of the ring-oxygen with sulphur or methylene, or replacement of a hydroxyl group with a halogen, an amino-, sulfhydryl-, or methyl group, and especially contemplated additions include methylene phosphonate groups. Further contemplated sugars also include sugar analogs (i.e., not naturally occurring sugars), and particularly carbocyclic ring systems. The term "carbocyclic ring system" as used herein refers to any molecule in which a plurality or carbon atoms form a ring, and in especially contemplated carbocyclic ring systems the ring is formed from 3, 4, 5, or 6 carbon atoms.

The term "nucleobase" or "base analog" refers to any heterocyclic base that is covalently coupled to a sugar, as described above. Examples of nucleobases include, but are not limited to, 5-fluorouracil, 5-chlorouracil, 5-bromouracil, 5-iodouracil, 5-trifluoromethyluracil, 5-vinyluracil, 5-bromovinyluracil, 2-amino-6-mercaptopurine, 6-methylpurine, 6-mercaptopurine, 2-fluoroadenine, 2-fluoro-6-aminopurine, *N*6,*N*6-dimethyladenine, *trans*-zeatin, thioguanine, 2-amino-6-bromopurine and 6-ethylthiopurine. More preferably, the nucleobase is 6-methylpurine, 6-mercaptopurine or 2-fluoroadenine.

The at least one further compound has complementary activities to the compound of formula (I) such that they do not adversely affect each other. The at least one further compound is suitably present in combination in amounts that are effective for the purpose intended.

The combination enhances the antiproliferative behaviour of compound I alone by providing "synergistic effects". Any of these effects may be attained when the active ingredients are: (1) co-formulated and administered or delivered simultaneously in a combined, unit dosage composition; (2) delivered by alternation or in parallel as separate compositions; or (3) by some other regimen. When delivered in alternation therapy, a synergistic effect may be attained when the compounds are administered or delivered sequentially.

As used herein, the terms "neoplastic cells", "neoplasia", "tumour", "tumour cells", "cancer" and "cancer cells" are used interchangeably and refer to cells which exhibit relatively autonomous growth, so that they exhibit an aberrant growth phenotype characterized by a significant loss of control of cell proliferation, and also includes malignant cells which can invade and destroy adjacent structures and/or metastasize. Neoplastic cells can be malignant or benign. In particular, the type cancer referred herein above and below is breast and prostate cancer. Cells that form metastases are "tumour cells", "neoplastic cells", and "cancer cells".

As used herein, the term "primary and metastatic breast cancer" relates to breast cancer cells that grow on breast (primary breast cancer) and also to breast cancer cells that have spread to distant sites (metastatic breast cancer). In the same sense, the term "primary and metastatic prostate cancer" relates to prostate cancer cells that grow in the prostate (primary prostate cancer) and also to a prostate cancer cells that have spread to distant sites (metastatic prostate cancer).

The terms "antineoplastic agent", "antineoplastic chemotherapeutic agent", "chemotherapeutic agent", "antineoplastic" and "chemotherapeutic" are used interchangeably and refer to chemical compounds or drugs which are used in the treatment or prevention of primary and metastatic cancer, e.g., to kill cancer cells and/or lessen the spread of the disease or decrease rate or size of metastases.

The terms "suppressing tumour growth", "treating tumour cell", and "treating cancer", and the like refer to reducing the rate of growth of a tumour/metastasis, halting tumour/metastasis growth completely, causing a regression in the size of an existing tumour/metastasis, eradicating an existing tumour/metastasis and/or preventing the occurrence of additional tumours/metastasis upon treatment with the active ingredient/s of the present disclosure. "Suppressing" tumour/metastasis growth indicates a growth state that is curtailed when compared to growth without administration of a compound disclosed herein. Tumour cell growth can be assessed by any mean known in the art, including, but not limited to, measuring tumour/metastasis size, determining whether tumour cells are proliferating using a ³H-thymidine incorporation assay, or counting tumour cells. "Suppressing" tumour cell growth means any or all of the following states: slowing, delaying, and stopping tumour growth, as well as tumour shrinkage.

The term "anti-proliferative agents," as used herein, refers to agents that modulate cell proliferation as defined herein.

"Modulating" cell proliferation of malignant cells means that the rate of proliferation is altered when compared to not administering an agent that interferes with tumour/metastasis growth. For purposes of this invention, "modulating" cell proliferation means that the rate of proliferation is decreased when compared to the rate of proliferation in that individual when no agent is administered, and includes any partial or total growth inhibition of the cells. However, during the course of therapy, for example, it may be desirable to increase the rate of proliferation from a previously measured level. In individuals afflicted with tumours/metastasis, the degree of modulation may be assessed by measurement of tumour cell proliferation, and generally entails detecting a proliferation marker(s) in a tumour cell population or uptake of certain substances which would provide a quantitative measure of proliferation. Any quantitative methods for measuring tumour cell proliferation currently known or unknown in the art can be used for this purpose. Further, it is possible that, if the cells are proliferating due to a genetic alteration (such as transposition, deletion, or insertion), this alteration could be detected using standard techniques in the art, such as RFLP (restriction fragment length polymorphism).

As used herein, "synergy" or "synergistic effect" when referring to combination of a compound of formula (I) of the present invention in conjunction with the at least one further compound means that the effect of the combination is more than additive when compared to the effect of the compound of formula (I) of the present invention alone.

The term "comprising" (and its grammatical variations) as used herein is used in the inclusive sense of "having" or "including" and not in the exclusive sense of "consisting only of".

When referred in the present invention to the term "and" in the context of "breast and prostate cancer", it should be understood that the compound of formula (I), combination or pharmaceutical composition according to the present invention can be used only for treating or preventing primary and metastatic breast cancer, only for treating or preventing primary and metastatic prostate cancer, or for treating or preventing both primary and metastatic breast and prostate cancer.

The term "pharmaceutically acceptable salts" of compound I as used herein, refers to pharmaceutically acceptable organic or inorganic salts of the compound of the invention. Exemplary salts include, but are not limited to, sulphate, citrate, acetate, oxalate, chloride, bromide, iodide, nitrate, bisulfate, phosphate, acid phosphate, isonicotinate, lactate, salycilate, acid citrate, tartrate, oleate, tannate, pantothenate, bitartrate, ascorbate, succinat, maleate, gentisinate, fumarate, gluconate, glucuronate, saccharate, formate, benzoate, glutamate, methanesulfonate "mesylate", ethanesulfonate, benzenesulfonate, p-toluenesulfonate, and pamoate salts. A pharmaceutically acceptable salt may have more than one charged atom in its structure. Instances where multiple charged atoms are part of the pharmaceutically acceptable salt can have multiple counter ions. Hence, a pharmaceutically acceptable salt can have one or more charged atoms and/or more counter ion. Chemical modification of a pharmaceutical compound into a salt is a technique well known to pharmaceutical chemists to obtain improved physical and chemical stability, hygroscopicity, flowability and solubility of compounds.

The phrase "pharmaceutically acceptable" indicates that the substance or composition must be compatible chemically and/or toxicologically, with the other ingredients comprising a formulation, and/or the mammal being treated therewith.

The compound of the present invention may exist in non-solvated as well as solvated forms with pharmaceutically acceptable solvents. A "solvate" or a "solvated form" refers to an association or complex of one or more solvent molecules and a compound of the invention. Examples of solvents that form solvates include, but are not limited to, water, isopropanol, ethanol, methanol, DMSO, ethyl acetate, acetic acid, and ethanolamine. The term "hydrate" refers to the complex where the solvent molecule is water.

The term "tautomer" or "tautomeric form" refers to structural isomer of different energies which are interconvertible via a low energy barrier. For example, proton tautomers (also known as prototropic tautomers) include interconversion via migration of a proton, such as keto-enol and imine-enamine isomerizations. Valence tautomers include interconversions by reorganization of some of the bonding electrons.

As used in the figures, the term "vehicle control" refers to the plain growth medium with DMSO added so that the final concentration of DMSO is the same than in all the control and test compound groups. In the experiments performed the final concentration of DMSO was 0.1%.

The present invention also relates to a pharmaceutical composition comprising the compound of formula (I) or a tautomer or a solvate or a hydrate or a pharmaceutically acceptable salt thereof as defined above as the active ingredient and a pharmaceutically acceptable carrier thereof for use in the treatment or prevention of primary and metastatic breast and prostate cancer. The present invention also relates to a pharmaceutical composition comprising the combination of the compound of formula (I) or a tautomer or a solvate or a hydrate or a pharmaceutically acceptable salt thereof with the at least one further compound or a pharmaceutically acceptable salt, acid or derivative thereof as defined above as the active ingredient and a pharmaceutically acceptable carrier thereof for use in the treatment or prevention of primary and metastatic breast and prostate cancer.

The present invention also relates to a method of prevention or treatment of primary and metastatic breast and prostate cancer in a subject comprising administering to said subject a therapeutically effective amount of the compound of formula (I) or a tautomer or a solvate or a hydrate or a pharmaceutically acceptable salt thereof. Preferably the subject is a human subject. In addition, the present invention also relates to a method of prevention or treatment of primary and metastatic breast and prostate cancer in a subject comprising administering to said subject a therapeutically effective amount of a combination of the compound of formula (I) or a tautomer or a solvate or a hydrate or a pharmaceutically acceptable salt thereof with the at least one further compound or a pharmaceutically acceptable salt, acid or derivative thereof as defined above. The present invention further relates to a method of prevention or treatment of primary and metastatic breast and prostate cancer in a subject comprising administering to said subject a therapeutically effective amount of a pharmaceutical composition as defined above.

The present invention also relates to the use of the compound of formula (I) or a tautomer or a solvate or a hydrate or a pharmaceutically acceptable salt thereof in the manufacture of a medicament for the prevention or treatment of primary and metastatic breast and prostate cancer. In addition, the present invention also relates to the use of a combination of the compound of formula (I) or a tautomer or a solvate or a hydrate or a pharmaceutically acceptable salt thereof with the at least one further compound or a pharmaceutically acceptable salt, acid or derivative thereof as defined above in the manufacture of a medicament for the prevention or treatment of primary and metastatic breast and prostate cancer. The present invention further relates to the use of a pharmaceutical composition as defined above in the manufacture of a medicament for the prevention or treatment of primary and metastatic breast and prostate cancer.

The compound of formula (I), the combination or the pharmaceutical composition as defined above may be administered for therapy by any suitable route including oral, rectal, nasal, topical (including buccal and sublingual), vaginal and parenteral (including subcutaneous, intramuscular, intravenous, intra-arterial, intrathecal, epidural and intradermal), transdermal, rectal, nasal, intraperitoneal, intrapulmonary and intranasal. It will be appreciated that the preferred route will vary with the condition and age of the recipient, the nature of the disease and the chosen active ingredient. Where the compound is administered orally, it may be formulated as a pill, capsule, tablet, etc. with a pharmaceutically acceptable carrier or excipient. Where the compound is administered parenterally, it may be formulated with a pharmaceutically acceptable parenteral vehicle and in a unit dosage injectable form.

The therapeutically effective amount or dosage of the compound, combination or pharmaceutical composition according to this invention can vary within wide limits and may be determined in a manner known in the art. Such dosage will be adjusted to the individual requirements in each particular case including the specific compound(s) being administered, the route of administration, the condition being treated, as well as the patient being treated. A dose may be administered once a day, twice per day, or more frequently, depending on the phamacodynamic properties, including absorption, distribution, metabolism, and excretion of the compound. In addition, toxicity factors may influence the dosage and administration regimen. When administered orally, the pill, capsule, or tablet may be ingested daily or less frequently for a specified period of time. The regimen may be repeated for a number of cycles of therapy.

The amounts required of the active ingredient will depend upon a number of factors including the severity of the condition to be treated and the identity of the recipient and will ultimately be at the discretion of the attendant physician or veterinarian. In general however, for each of these utilities and indications, a suitable, but not limited thereto, effective dose of the active ingredient will be in the range of 0.001 to 100 mg per kilogram body weight of recipient per day.

While it is possible for the active ingredient to be administered alone, it is preferable to present it as a pharmaceutical composition.

In the context of the present invention, each carrier must be "acceptable" in the sense of being compatible with the other ingredients of the composition and not deleterious to the recipient thereof.

The compound of formula (I), the combination and pharmaceutical composition according to the present invention may conveniently be presented in unit dosage form prepared in accordance with standard pharmaceutical practice as pharmaceutical compositions. Methods used in the art of pharmacy include the step of bringing into association the active ingredient with the carrier which constitutes one or more accessory ingredients. In general, the compositions are prepared by uniformly and intimately bringing into association the active ingredient with liquid carriers or finely divided solid carrier or both, excipients, and then, if necessary, shaping the product. Suitable carriers, diluents and excipients are well known to those skilled in the art and include materials such as carbohydrates, waxes, water soluble and/or swellable polymers, hydrophilic or hydrophobic materials, gelatine, oils, solvents, water and the like. The formulations may also include one or more buffers, stabilizing agents, surfactants, wetting agents, lubricating agents, emulsifiers, suspending agents, preservatives, antioxidants, opaquing agents, glidants, processing aids, colorants, sweeterners, perfuming agents, flavouring agents and other known additives to provide an elegant presentation of the drug (i.e. compound of formula (I), combination or pharmaceutical composition according to the present invention) or aid in the manufacturing of the pharmaceutical product (i.e. the medicament).

The compound of formula (I), the combination or pharmaceutical composition according to the present invention suitable for oral administration may be presented as discrete units such as capsules, cachets, sachets of granules or tablets (such as a swallowable, dispersible or chewable tablet) each containing a predetermined amount of the active ingredient; as a powder of granules; as a solution or a suspension in an aqueous liquid or a non-aqueous liquid; or as an oil-in-water liquid emulsion or a water-in-oil liquid emulsion. The active ingredient may also be present as a bolus, electuary or paste.

A tablet may be made by compression or moulding optionally with one or more accessory ingredients. Compressed tablets may be prepared by compressing in a suitable machine the active ingredient in a free-flowing form such as a powder of granules, optionally mixed with a binder, lubricant, inert diluent, preservative, surface active or dispersing agent. Moulded tablets may be made by moulding in a suitable machine a mixture of the powdered compound moistened with an inert liquid diluent. The tablets may optionally be coated or scored any may be formulated so as to provide slow or controlled release of the active ingredient therein.

The compound of formula (I), the combination or pharmaceutical composition according to the present invention suitable for topical administration in the mouth include lozenges comprising the active ingredient in a flavoured basis, usually sucrose and acacia or tragacanth; pastilles comprising the active ingredient in an inert basis such as gelatine and glycerine, or sucrose and acacia; and mouthwashes comprising the active ingredient in suitable liquid carrier.

The compound of formula (I), the combination or pharmaceutical composition according to the present invention for rectal administration may be present as a suppository with a suitable base comprising or example cocoa butter or a salicylate.

The compound of formula (I), the combination or pharmaceutical composition according to the present invention suitable for vaginal administration may be present as pessaries, tampons, creams, gels, pastes, foams or spray formulations containing in addition to the active ingredient such carriers as are known in the art to be appropriate.

The compound of formula (I), the combination or pharmaceutical composition according to the present invention suitable for parenteral administration include aqueous and non-aqueous isotonic sterile injection solution which may contain antioxidants, buffers, bacteriostats and solutes which render the formulation aqueous and non-aqueous sterile suspensions which may include suspending agents and thickening agents. The compositions may be presented in unit-dose or multidose sealed containers, for example, ampoules and vial, and may be stored in a freeze-drier (lyophilized) condition requiring only the addition of the sterile liquid carrier, for example water for injections, immediately prior to use. Extemporaneous injection solutions and suspensions may be prepared from sterile powders, granules and tablets of the kind previously described.

The active ingredient may also be presented in a composition comprising micrometre- or nanometre-size particles of active ingredient.

It should be understood that in addition to the ingredients particularly mentioned above the composition of this invention may include other agents conventional in the art having regard to the type of formulation in question, for example those suitable for oral administration may include flavouring agents or taste masking agents.

The compound of formula (I), the combination or pharmaceutical composition according to the present invention may be packaged in a variety of ways depending upon the method used for administering the drug. Generally, an article for distribution includes a container having deposited therein the compound of formula (I), the combination or pharmaceutical composition according to the present invention in an appropriate form. Suitable containers are well known to those skilled in the art, and include materials such as bottles (plastic and glass), sachets, ampoules, plastic bags, metal cylinders, and the like. The container may also include a tamper-proof assemblage to prevent indiscreet access to the contents of the package. In addition, the container has deposited thereon a label that describes the contents of the container. The label may also include appropriate warnings.

The compound of formula (I), the combination or pharmaceutical composition according to the present invention ordinarily can be stored as a solid composition, a lyophilized formulation or as an aqueous solution.

In a particular embodiment of breast and prostate cancer therapy, the compound of formula (I), the combination or the pharmaceutical composition as defined in the present invention are used in the treatment or prevention of primary and metastatic breast and prostate cancer combined with surgical therapy and/or radiotherapy.

Compound of formula (I) may be synthesized from 4-chloropyrazolo[3,4-*d*]pyrimidine by aqueous or alcoholic ammonia, or from 3-amino-4-cyanopyrazole by formamide treatment (Robins *et al,* supra).

The following Examples are offered for illustrative purposes only and are not intended to limit the scope of the present invention in any way.

### EXAMPLES

Determination of the ability of compound of formula (I) and combinations between compound of formula (I) and certain therapeutic compounds described herein to inhibit the proliferation of target cells was assayed *in vitro* against breast and prostate tumour cells. The antiproliferative activity was measured through the commercial WST-1 proliferation measurement reagent in a two-step screening procedure: (1) the target cell lines (MCF-7, LNCaP and PNT1A) were exposed to only one strong concentration of samples (1 microM) for five days before measurement of proliferation and the result of each samples was compared against the same concentration of reference compounds (Gemcitabine, Floxuridine, Cytarabine); (2) for those compounds where primary test revealed certain effects, the target cell lines were exposed to three concentration of compound (1 micromolar, 100 nanomolar and 10 nanomolar) and the proliferation of cells was measured in three time points (1 day, 2 days and 5 days) in order to draw a growth curve for every tested sample and control compound.

Compound of formula (I) and exemplary combinations of compound of formula (I) and at least one further compound (combinations A to K in Table 1) were prepared and tested for antiproliferative activity according to the methods of this invention, and have the following structures and corresponding names.

**Table 1**

| **Compound** | **Structure** | **Name** |
|---|---|---|
| **I** | 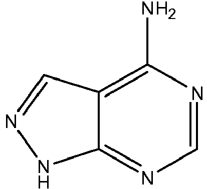 | 4-Aminopyrazolo[3,4-*d*]pyrimidine |
| **A** | 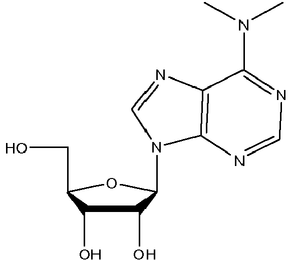 | *N*6,*N*6-Dimethyladenosine |
| **B** | 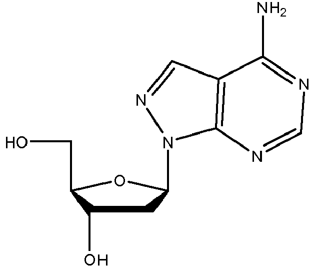 | 8-Aza-7-deaza-2'-deoxyadenosine |
| **C** | 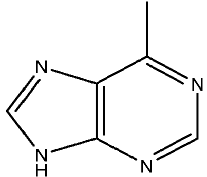 | 6-Methylpurine |
| **D** | 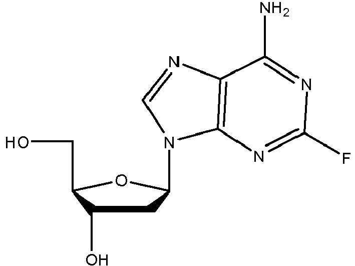 | 2-Fluoro-2'-deoxyadenosine |
| **E** | 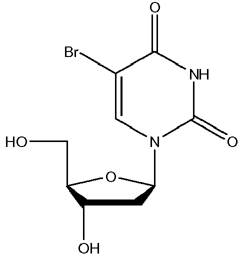 | 5-Bromo-2'-deoxyuridine (Broxuridine) |
| **F** | 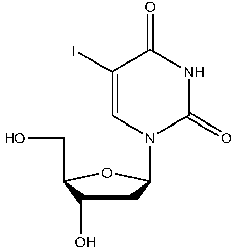 | 5-Iodo-2'-deoxyuridine (Idoxuridine) |
| **G** | 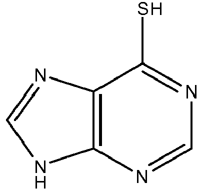 | 6-Mercaptopurine |
| **H** | 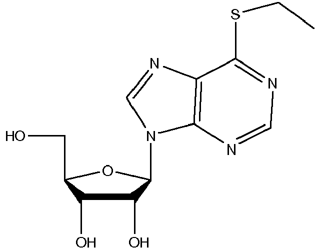 | 6-(Ethylthio)inosine |
| **J** | 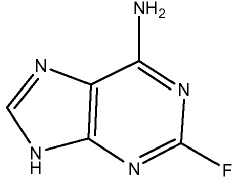 | 2-Fluoroadenine |
| **K** | 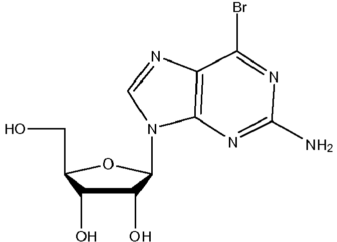 | 2-(2-Amino-6-bromopurin-9-yl)-5-(hydroxymethyl)oxolane-3,4-diol |
| **L** | 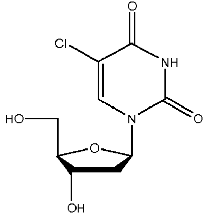 | 5-Chloro-2'-deoxyuridine |

### Example 1: In vitro cell proliferation assay

### Description of Tumor Cell Lines and Cultures:

MCF-7 and LNCaP cells were purchased from ATCC. PNT1A cells were purchased from ECACC. The cells were grown in a subconfluent condition to maintain a logarithmic growth phase. The cells were dislodged for passage using trypsin-EDTA.

### Tetrazolium dye proliferation (WST-1) assay:

The proliferation of the cell lines was evaluated by using the commercial WST-1 proliferation measurement reagent. Cells from exponentially growing cultures were plated on the 96 well plates at the appropriate seeding density in the optimal medium to give logarithmic growth over the course of the assay. The plates with MCF-7 and PNT1A cells were incubated 24h at 37°C in a humidified CO₂ atmosphere to allow for recovery from trypsinization before adding of test and control compounds. The plates containing LNCaP cells were incubated 48h at 37°C in a humidified CO₂ atmosphere before the adding of test and control compounds. 1mM stock solutions were prepared from test compounds in DMSO (dimethylsulfoxide) and stored at -20°C. Control compounds Ara-C and Gemcitabine stock solutions were prepared in sterile water. Each test and control compound was diluted twice the final concentration in appropriate media. Half of the final well volume of each dilution was added in six parallels to plates containing cells. Required volume of DMSO was added into the vehicle and control compound dilutions to achieve the final concentration of 0.1% DMSO in all control and test compound wells. The plates were returned to 37°C in a humidified CO₂ atmosphere and incubated for 5 days. WST-1 reagent (2-(4-Iodophenyl)-3-(4-nitrophenyl)-5-(2,4-disulfophenyl)-2H-tetrazolium) was added to each well according manufacturer's instructions and the plates were returned to 37°C in a humidified CO₂ atmosphere for 2 hours. Following incubation the absorbance at a wavelength of 450nm was measured from each well using a multilabel counter. The absorbance at wavelength 450nm correlates linearly to number of viable cells in the well.

The means and standard deviations for parallel samples were calculated and plotted as a graph.

### Results

The antiproliferative activity of compound of formula (I) was evaluated *in vitro* using tetrazolium dye proliferation assay in different cell lines: MCF-7 (breast cancer cell line, derived from pleura fluid), LNCaP (prostate cancer cell line, derived from lymph node metastasis) and PNT1A (normal prostate cell line, SV40 immortalized). Table 2 below shows the results obtained for compound of formula (I) in comparison with control compounds Ara-C, Gemcitabine and Floxuridine. The *in vitro* studies demonstrate superior antiproliferative activity of compound of formula (I) in breast and prostate cancer cell lines and minor antiproliferative effect of compound of formula (I) in normal non tumour prostate cell line.

**Table 2. The proliferation inhibitory effect of compound I and control nucleoside analogs Ara-C, Gemcitabine and Floxuridine in MCF-7 breast cancer cell line, LNCaP prostate cancer cell line and PNT1A immortalized normal prostate cell line compared with 0.1 % DMSO vehicle control group.**

| **Cell line** | **Ara-C** | **Gemcitabine** | **Floxuridine** | **Compound I** |
|---|---|---|---|---|
| **MCF-7** | 78.3±3.9% | 80.0±3.7% | 66.2±8.0% | 98.6±0.5% |
| **LNCaP** | 50.1±3.4% | 71.2±0.8% | 58.3±1.0% | 97.9±0.5% |
| **PNT1A** | 66.5±1.8% | 98.8±0.3% | 88.3±0.6% | 18.0±2.1% |

Tables 3 to 12 below show the results obtained for selected combinations of compound of formula (I) and exemplarized further compound in comparison with control compounds Gemcitabine and Floxuridine. The *in vitro* studies demonstrate superior antiproliferative activity of compound of combinations containing compound of formula (I) in breast and prostate cancer cell lines.

**Table 3. The proliferation inhibitory effect of compounds I and A, combination of compounds I and A, and control nucleoside analog Floxuridine in MCF-7 breast cancer cell line compared with Gemcitabine. Increase of the effect of the combination compared with Gemcitabine.**

| **Cell line** | **Gemcitabine** | **Floxuridine** | **Compound I** | **Compound A** | **Compounds I + A** | **Inhibition increase** |
|---|---|---|---|---|---|---|
| **MCF-7** | 0 | -50.4±35.6% | 93.8±2.4% | 62.6±9.4% | 98.6±2.2% | 5% |

**Table 4. The proliferation inhibitory effect of compounds I and B, combination of compounds I and B and control nucleoside analog Floxuridine in MCF-7 breast cancer cell line compared with Gemcitabine. Increase of the effect of the combination compared with Gemcitabine.**

| **Cell line** | **Gemcitabine** | **Floxuridine** | **Compound I** | **Compound B** | **Compounds I+B** | **Inhibition increase** |
|---|---|---|---|---|---|---|
| **MCF-7** | 0 | -50.4±35.6% | 93.8±2.4% | -317.1±15.0% | 94.4±2.6% | 0.6% |

**Table 5. The proliferation inhibitory effect of compounds I and C, combination of compounds I and C and control nucleoside analog Floxuridine in MCF-7 breast cancer cell line and LNCaP prostate cancer cell line compared with Gemcitabine. Increase of the effect of the combination compared with Gemcitabine.**

| **Cell line** | **Gemcitabine** | **Floxuridine** | **Compound I** | **Compound C** | **Compounds I + C** | **Inhibition increase** |
|---|---|---|---|---|---|---|
| **MCF-7** | 0 | -50.4±35.6% | 93.8±2.4% | 65.9±7.6% | 97.1±2.4% | 3.5% |
| **LNCaP** | 0 | -34.7±3.1% | 93.1±0.7% | 86.9±1.6% | 97.1±0.7% | 4.2 % |

**Table 6. The proliferation inhibitory effect of compounds I and D, combination of compounds I and D and control nucleoside analog Floxuridine in MCF-7 breast cancer cell line and LNCaP prostate cancer cell line compared with Gemcitabine. Increase of the effect of the combination compared with Gemcitabine.**

| **Cell line** | **Gemcitabine** | **Floxuridine** | **Compound I** | **Compound D** | **Compounds I + D** | **Inhibition increase** |
|---|---|---|---|---|---|---|
| **MCF-7** | 0 | -50.4±35.6% | 93.8±2.4% | -88.2±49.0% | 95.1±2.8% | 1.3% |
| **LNCaP** | 0 | -34.7±3.1% | 93.1±0.7% | 57.0±1.0% | 97.0±1.2% | 4.2% |

**Table 7. The proliferation inhibitory effect of compounds I and E, combination of compounds I and E and control nucleoside analog Floxuridine in MCF-7 breast cancer cell lines compared with Gemcitabine. Increase of the effect of the combination compared with Gemcitabine.**

| **Cell line** | **Gemcitabine** | **Floxuridine** | **Compound I** | **Compound E** | **Compounds I + E** | **Inhibition increase** |
|---|---|---|---|---|---|---|
| **MCF-7** | 0 | -50.4±35.6% | 93.8±2.4% | -221.1±50.3% | 97.3±1.4% | 3.7% |

**Table 8. The proliferation inhibitory effect of compounds I and F, combination of compounds I and F and control nucleoside analog Floxuridine in MCF-7 breast cancer cell line compared with Gemcitabine. Increase of the effect of the combination compared with Gemcitabine.**

| **Cell line** | **Gemcitabine** | **Floxuridine** | **Compound I** | **Compound F** | **Compounds I + F** | **Inhibition increase** |
|---|---|---|---|---|---|---|
| **MCF-7** | 0 | -50.4±35.6% | 93.8±2.4% | -174.8±33.9% | 98.1±1.8% | 4.6% |

**Table 9. The proliferation inhibitory effect of compounds I and G, combination of compounds I and G and control nucleoside analog Floxuridine in MCF-7 breast cancer cell line compared with Gemcitabine. Increase of the effect of the combination compared with Gemcitabine.**

| **Cell line** | **Gemcitabine** | **Floxuridine** | **Compound I** | **Compound G** | **Compounds I + G** | **Inhibition increase** |
|---|---|---|---|---|---|---|
| **MCF-7** | 0 | -50.4±35.6% | 93.8±2.4% | -105.8±36.8% | 99.4±2.7% | 5.9% |

**Table 10. The proliferation inhibitory effect of compounds I and H, combination of compounds I and H and control nucleoside analog Floxuridine in MCF-7 breast cancer cell line and LNCaP prostate cancer cell line compared with Gemcitabine. Increase of the effect of the combination compared with Gemcitabine.**

| **Cell line** | **Gemcitabine** | **Floxuridine** | **Compound I** | **Compound H** | **Compounds I and H** | **Inhibition increase** |
|---|---|---|---|---|---|---|
| **MCF-7** | 0 | -50.4±35.6% | 93.8±2.4% | -45.5±10.5% | 99.9±1.5% | 6.5% |
| **LNCaP** | 0 | -34.7±3.1% | 93.1±0.7% | -342.7±41.7% | 94.9±0.7% | 2.0% |

**Table 11. The proliferation inhibitory effect of compounds I and J, combination of compounds I and J and control nucleoside analog Floxuridine in LNCaP prostate cancer cell line compared with Gemcitabine. Increase of the effect of the combination compared with Gemcitabine.**

| **Cell line** | **Gemcitabine** | **Floxuridine** | **Compound I** | **Compound J** | **Compounds I + J** | **Inhibition increase** |
|---|---|---|---|---|---|---|
| **LNCaP** | 0 | -34.7±3.1% | 93.1±0.7% | 96.4±0.9% | 98.3±1.1% | 5.6% |

**Table 12. The proliferation inhibitory effect of compounds I and K, combination of compounds I and K and control nucleoside analogs Ara-C and Floxuridine in LNCaP prostate cancer cell line compared with Gemcitabine. Increase of the effect of the combination compared with Gemcitabine.**

| **Cell line** | **Gemcitabine** | **Floxuridine** | **Compound I** | **Compound K** | **Compounds I+K** | **Inhibition increase** |
|---|---|---|---|---|---|---|
| **LNCaP** | 0 | -34.7±3.1% | 93.1±0.7% | -295.8±52.8% | 96.8±1.5% | 4.0% |

**Table 13. The proliferation inhibitory effect of compounds I and L, combination of compounds I and L and control nucleoside analogs Ara-C and Floxuridine in LNCaP prostate cancer cell line compared with Gemcitabine. Increase of the effect of the combination compared with Gemcitabine.**

| **Cell line** | **Gemcitabine** | **Floxuridine** | **Compound I** | **Compound L** | **Compounds I + L** | **Inhibition increase** |
|---|---|---|---|---|---|---|
| **LNCaP** | 0 | -34.7±3.1% | 93.1±0.7% | -281.4±36.4% | 96.0±0.8% | 3.1% |

Table 14 below shows the results obtained for selected combinations of compound of formula (I) and exemplified a further compound in comparison with control compounds Gemcitabine and Floxuridine. The *in vitro* studies demonstrate a lower antiproliferative activity of selected combinations of compound of formula (I) in normal non tumour prostate cell line than reference compound Gemcitabine.

**Table 14. The proliferation inhibitory effect of combination of compound I and exemplary therapeutic compounds, control nucleoside analogs Ara-C, gemcitabine and Floxuridine in PNT1A immortalized normal prostate cell line compared with 0.1 % DMSO vehicle control group ^{(a)}. ^{(b)} Decrease in the antiproliferation inhibitory effect of combinations of compound I and exemplary therapeutic compounds in PNT1A immortalized normal prostate cell line compared with Gemcitabine.**

| **Combination compounds** | **Ara-C^{(a)}** | **Gemcitabine^{(a)}** | **Floxuridine^{(a)}** | **Antiproliferation effect of Combinations^{(a)}** | **Inhibition decrease^{(b)}** |
|---|---|---|---|---|---|
| **I+A** | 66.5±1.8% | 98.8±0.3% | 88.3±0.6% | 17.2±4.0% | -82.6% |
| **I+B** | 66.5±1.8% | 98.8±0.3% | 88.3±0.6% | 15.4±6.5% | -84.4% |
| **I+C** | 66.5±1.8% | 98.8±0.3% | 88.3±0.6% | 51.0±4.2% | -48.4% |
| **I+D** | 66.5±1.8% | 98.8±0.3% | 88.3±0.6% | 97.3±0.4% | -1.5% |
| **I+E** | 66.5±1.8% | 98.8±0.3% | 88.3±0.6% | 26.2±6.7% | -73.5% |
| **I+F** | 66.5±1.8% | 98.8±0.3% | 88.3±0.6% | 38.6±5.7% | -60.9% |
| **I+G** | 66.5±1.8% | 98.8±0.3% | 88.3±0.6% | 49.8±1.6% | -49.6% |
| **I+H** | 66.5±1.8% | 98.8±0.3% | 88.3±0.6% | 50.9±1.9% | -48.5% |
| **I+J** | 66.5±1.8% | 98.8±0.3% | 88.3±0.6% | 98.4±0.2% | -0.4% |
| **I+K** | 66.5±1.8% | 98.8±0.3% | 88.3±0.6% | 21.1±4.3% | -78.6% |
| **I+L** | 66.5±1.8% | 98.8±0.3% | 88.3±0.6% | 47.4±1.8% | -52.0% |

## Claims

1. Compound of formula (I) 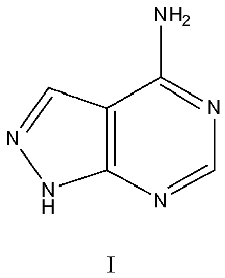 or a tautomer, solvate, hydrate or a pharmaceutically acceptable salt thereof for use in the treatment or prevention of primary and metastatic breast cancer.

2. Compound of formula (I) 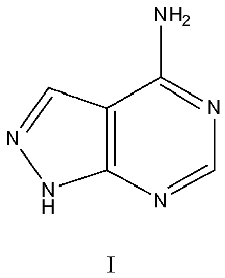 or a tautomer, solvate, hydrate or a pharmaceutically acceptable salt thereof for use in the treatment or prevention of primary and metastatic prostate cancer.

3. Combination of a compound of formula (I) 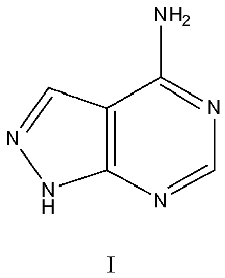 or a tautomer, solvate, hydrate or a pharmaceutically acceptable salt thereof with at least one further compound or a pharmaceutically acceptable salt, acid or derivative thereof for use in the treatment or prevention of primary and metastatic breast cancer.

4. Combination of a compound of formula (I) 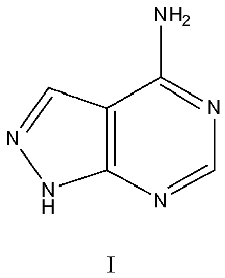 or a tautomer, solvate, hydrate or a pharmaceutically acceptable salt thereof with at least one further compound or a pharmaceutically acceptable salt, acid or derivative thereof for use in the treatment or prevention of primary and metastatic prostate cancer.

5. Combination according to claim 3 or 4, wherein said at least one further compound includes a chemotherapeutic agent, such as an alkylating agent, antimetabolites (including nucleoside analogs and nucleobases), spindle poison plant alkaloid, cytotoxic/antitumour antibiotic, topoisomerase inhibitor, antibody, photosensitizer, kinase inhibitor; an anti-hormonal agent; an aromatase inhibitor; an anti-androgen; a ribozyme; a vaccine such as gene therapy vaccine; an anti-angiogenic agent; or an antiviral agent.

6. Combination according to claim 5, wherein said at least one further compound is a chemotherapeutic agent selected from 5-fluorouracil, gemcitabine, paclitaxel capecitabine, cis-platin, mitoxantrone, etoposide, camptothecin, vinblastine, paclitaxel, docetaxel, mithramycin A, dexamethasone, caffeine and pharmaceutically acceptable salts, acids or derivatives thereof.

7. Combination according to claim 5, wherein said at least one further compound is a nucleoside analog or a nucleobase.

8. Combination according to claim 7, wherein said at least one further compound is a nucleoside analog selected from 5-fluoro-2'-deoxyuridine, 5-chloro-2'-deoxyuridine, 5-bromo-2'-deoxyuridine, 5-iodo-2'-deoxyuridine, 5-fluorouridine, 5-chlorouridine, 5-bromouridine, 5-iodouridine, 6-methylpurine-2'-deoxyriboside, 6-methylpurine riboside, 2-fluoro-2'-deoxyadenosine, 2-fluoroadenosine, trifluorothymidine, 5-bromovinyl-2'-deoxyuridine, *N*6, *N*6-dimethyladenosine, 6-mercaptoguanosine, 8-aza-7-deaza-2'-deoxyadenosine, *trans*-zeatin riboside, *trans*-zeatin deoxyriboside, 5-vinyl-2'-deoxyuridine, 2-amino-6-bromopurine riboside, 2-amino-6-bromopurine deoxyriboside or 6-(ethylthio)inosine.

9. Combination according to claim 7, wherein said at least one further compound is a nucleobase selected from 5-fluorouracil, 5-chlorouracil, 5-bromouracil, 5-iodouracil, 5-trifluoromethyluracil, 5-vinyluracil, 5-bromovinyluracil, 2-amino-6-mercaptopurine, 6-methylpurine, 6-mercaptopurine, 2-fluoroadenine, 2-fluoro-6-aminopurine, *N*6, *N*6-dimethyladenine, *trans*-zeatin, thioguanine, 2-amino-6-bromopurine or 6-ethylthiopurine.

10. Combination according to claim 8, wherein said at least one further compound is a nucleoside analog selected from 5-bromo-2'-deoxyuridine, 5-iodo-2'-deoxyuridine, 2-fluoro-2'-deoxyadenosine, *N*6,*N*6-dimethyladenosine, 8-aza-7-deaza-2'-deoxyadenosine, 2-amino-6-bromopurine deoxyriboside or 6-(ethylthio)inosine.

11. Combination according to claim 9, wherein said at least one further compound is a nucleobase selected from 6-methylpurine, 6-mercaptopurine or 2-fluoroadenine.

12. Pharmaceutical composition comprising the compound according to claim 1 or 2 or the combination according to any of claims 3 to 11 for use in the treatment or prevention of primary and metastatic breast and prostate cancer.

13. Compound according to claim 1 or 2, combination according to any of claims 3 to 11 or pharmaceutical composition according to claim 12 which is administered by oral, rectal, nasal, topical, vaginal, parenteral, transdermal, rectal, nasal, intraperitoneal, intrapulmonary and intranasal route.

14. Compound according to claim 1 or 2, combination according to any of claims 3 to 11 or pharmaceutical composition according to claim 12, for use in the treatment or prevention of primary and metastatic breast cancer combined with surgical therapy and/or radiotherapy.

15. Compound according to claim 1 or 2, combination according to any of claims 3 to 11 or pharmaceutical composition according to claim 12, for use in the treatment or prevention of primary and metastatic prostate cancer combined with surgical therapy and/or radiotherapy.
